Europäisches Patentamt

(19) European Patent Office

Office européen des brevets

(11) Publication number: **0 105 996**
**A1**

# (12) EUROPEAN PATENT APPLICATION

(21) Application number: **82401896.4**

(22) Date of filing: **15.10.82**

(51) Int. Cl.³: **A 61 K 31/13, A 61 K 31/41**

(43) Date of publication of application: **25.04.84**
Bulletin 84/17

(84) Designated Contracting States: **AT BE CH DE FR GB IT LI LU NL SE**

(71) Applicant: **MERCK & CO. INC., 126, East Lincoln Avenue P.O. Box 2000, Rahway New Jersey 07065 (US)**

(72) Inventor: **Lotti, Victor J., 214 Brookside Circle, Harleysville Pennsylvania 19438 (US)**

(74) Representative: **Martin, Jean-Jacques et al, Cabinet REGIMBEAU 26, Avenue Kléber, F-75116 Paris (FR)**

(54) Ophthalmic compositions for treating elevated intraocular pressure.

(57) This invention relates to compositons of the R stereoisomeric form of β-adrenergic blocking agents that are topically effective in the treatment of elevated intraocular pressure. More particularly, it relates to compositions comprising the R stereoisomeric form of β-adrenergic blocking agents which are enriched with respect to the S stereoisomer thereof so that when applied topically to the eye there results a decreased intraocular pressure. In particular, this invention relates to compositions of (R)-(+)-1-tert-butylamino-3- [(4-morpholino-1,2,5-thiazol-3-yl)-oxy]- 2-propanol and ophthalmologically acceptable acid addition salts thereof and their use to lower intraocular pressure, especially in the treatment of ocular hypertension and glaucoma.

EP 0 105 996 A1

- 1 -  16618

## TITLE OF THE INVENTION
Ophthalmic Compositions for Treating Elevated
Intraocular Pressure

## DISCLOSURE OF THE INVENTION

This invention relates to compositions of the R stereoisomeric form of β-adrenergic blocking agents that are topically effective in the treatment of elevated intraocular pressure. More particularly, it relates to compositions comprising the R stereoisomeric form of β-adrenergic blocking agents which are enriched with respect to the S stereoisomer thereof so that when applied topically to the eye there results a decreased intraocular pressure. In particular, this invention relates to compositions of (R)-(+)-1-tert-butylamino-3-[(4-morpholino-1,2,5-thiazol-3-yl)oxy]-2-propanol and ophthalmologically acceptable acid addition salts thereof and their use to lower intraocular pressure, especially in the treatment of ocular hypertension and glaucoma.

Glaucoma is an optic neuropathy associated with elevated ocular pressures which are too high for normal function and result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Indeed, few advances were made in the treatment of glaucoma since pilocarpine and physostigmine were introduced. Recently clinicians have noted that many β-adrenergic blocking agents were effective in reducing intraocular pressure. While many of these were effective in reducing intraocular pressure they also had other characteristics, e.g. membrane stabilizing activity that were not acceptable for chronic ocular use. S-(-)-1-tert-butylamino-3-[(4-morpholino-1,2,5-thiadiazol-3-yl)oxy]-2-propanol, a β-adrenergic blocking agent, was found to reduce intraocular pressure and be devoid of many unwanted side effects associated with pilocarpine and in addition possess advantages over many other β-adrenergic blocking agents, e.g. lack of local anesthesia, long duration of activity, minimal tolerance etc. It was nevertheless found that S-(-)-1-tert-butyl amino-3-[(4-morpholino-1,2,5-thiadiazol-3-yl)oxy]-2-propanol, must be used with caution in those few patients that have elevated intraocular pressure and also suffer from bronchial asthma, sinus bradycardia and greater than first degree block; cardiogenic shock, right ventricular failure secondary to pulmonary hypertension; or congestive heart failure; and its concommitant use with adrenergic augmenting cyclotropic drugs must be carefully monitored. These precautions are necessary because even when administered topically to the eye the β-adrenergic blocking agent is sufficiently active that a small portion is absorbed into the systemic circulation where it can effect other systems. The isomeric form of the β-adrenergic blocking agents that has the R configuration, although considerably less active than the S configuration in most other systems outside the eye, nevertheless exhibits in the eye a nearly equivalent degree of activity. This discovery is contrary to the initial belief that in order to achieve full activity in the eye a β-adrenergic blocking agent needed to be the S stereoisomeric form, the form that was most active in other extraocular systems. Indeed, the commercially sold form of 1-tert-butylamino-3-[(4-morpholino-1,2,5-thiadiazol-3-yl)oxy]-2-propanol is the S-configuration since this was believed to be the maximally active form. Quite startlingly, the eye has proven to respond differently to the R configuration of timolol than most other extraocular systems. Therefore to obtain an intraocular pressure reducing response in the eye one does not require that there be employed a racemate or the β-adrenergic blocking agent enriched in isomers of the S-configuration .

This discovery means that in those patients who have a history of asthma or congestive heart disease and for whom timolol was either contraindicated or necessarily prescribed with caution, can now benefit from this revolutionary form of glaucoma therapy. This is because such patients can receive the β-adrenergic blocking agent enriched in the R isomer with respect to the S isomer or more preferably the R form substantially free of S isomer. It further means that those β-adrenergic blocking agents that had side effects on extraocular systems that contraindicated their use in the eye may also now be used for the treatment of glaucoma since most other extraocular systems will be only minimally affected by the topical administration into the eye of the relatively inactive R form of the β-adrenergic blocking agent.

The term "substantially free" as used herein means the isomer indicated in better than 85% purity, and in those instances where resolution techniques permit better than 90, and even 95% purity and better, with respect to other isomers.

The R-isomer of the β-adrenergic blocking agent substantially free of the S isomer is preferably administered in the form of ophthalmic pharmaceutical compositions adapted for topical administration to the eye such as solutions, ointments or as a solid insert. In the rabbit, a 1% solution of R-(+)-3-tert-butylamino-3-[(4-morpholino-1,2,5-thiadiazol-3-yl)oxy]-2-propanol in a normal saline solution was found effective in lowering intraocular pressure. For other species of animal and man, the dose must be adjusted accordingly.

Generally, in man the dose must be patient adjusted to employ the minimal dose that reduces intraocular pressure to an acceptable level. In general, formulations of this invention may contain from 0.01 to 5% and especially 0.5 to 2% of medicament. Higher dosages as, for example, about 10% or lower dosages can be employed provided the dose is effective in lowering intraocular pressure. As a unit dosage form between 0.001 to 5.0 mg., preferably .005 to 2.0 mg., and especially 0.005 to 1.0 mg. of the compound is generally applied to the human eye.

Those β-adrenergic blocking agents that may advantageously have their extraocular tissue activity minimized by topically applying to the eye the R stereoisomeric form are those having a stereoisomeric center and include: methypranol; propranolol; alprenolol; oxprenolol; practolol; acebutolol; prindolol; metoprolol; atenolol; sotalol; metindol; nadolol; labetalol; practolol; bunitrolol; RMI 81968 (medroxalol); tiprenolol; tolamolol; celiprolol; mepindolol; indenolol; bupranolol; penbutolol; trimepranol; (R)-2-(3-(1,1-dimethylethyl)-amino-2-hydroxypropoxy)-3-pyridenecarbonitrile hydrochloride; 1-tertbutylamino-3-(2,5-dichloro-phenoxy)-2-propanol; 1-isopropylamino-3-[4-(2-cyclo-propylmethoxy-ethyl)phenoxy]-2-propanol; 3-isopropyl-amino-1-(7-methylindan-4-yloxy)-2-butanol; 2-(3-tert-butylamino-2-hydroxypropylthio)-4-(5-carbamoyl-2-thienyl)thiazole, and 7-(2-hydroxy-3-tert-butylamino-propoxy)-phthalide, gutolol, SL 75212, ICI 118551, GYKI 41099, DL 071-IT, BFE-60, S-596, and MK-761.

The R isomer can be separated from the racemate by means known in the art.

The thrust of this invention as hereinbefore stated is to provide a ophthamologically active anti-hypertensive agent for the eye, both human and animal, that avoids unwanted effects on extraocular tissues. With the discovery that the R form of a β-adrenergic beta blocking agent is active in the eye, and at the same time is relatively inactive, compared to the S isomer, in extraocular tissue, it becomes possible to administer a β- adrenergic blocking agent that is enriched in the R isomer with respect to the S isomer, and avoid initiation of undesireable extraocular effects. Of course to obtain the maximum freedom from extraocular side reactions R isomer substantially free from S isomer should be administered. Thus a pharmaceutic dosage is suitable where as compared to a β-adrenergic blocking agent that is substantially S, an entity is employed where the R isomer is enriched with respect to the S.

The pharmaceutical preparation which contains the compound may be conveniently admixed with a non-toxic pharmaceutical organic carrier, or with a non-toxic pharmaceutical inorganic carrier. Typical of pharmaceutically acceptable carriers are, for example, water, mixtures of water and water-miscible solvents such as lower alkanols or aralkanols, vegetable oils, polyalkylene glycols, petroleum based jelly, ethyl cellulose, ethyl oleate, carboxymethylcellulose, polyvinylpyrrolidone, isopropyl myristate and other conventionally employed acceptable carriers. The pharmaceutical preparation may also contain non-toxic auxiliary substances such as emulsifying, preserving, wetting agents, bodying agents and the like, as for example, polyethylene glycols 200, 300, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, methyl and propyl paraben, benzyl alcohol, phenyl ethanol, buffering ingredients such as sodium chloride, sodium borate, sodium acetates, gluconate buffers, and other conventional ingredients such as sorbitan monolaurate, triethanolamine, oleate, polyoxyethylene sorbitan monopalmitylate, dioctyl sodium sulfosuccinate, monothioglycerol, thiosorbitol, ethylenediamine tetracetic acid, and the like. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles and the like. The pharmaceutical preparation may also be in the form of a solid insert.

While many patients find liquid medication to be entirely satisfactory, others may prefer a solid medicament that is topically applied to the eye, for example, a solid dosage form that is suitable for insertion into the cul-de-sac. To this end the R form of the β adrenergic blocking agent can be included with a non-bioerodible insert, i.e. one which after dispensing the drug remains essentially intact, or a bioerodible insert, i.e. one that either is soluble in lacrimal fluids, or otherwise disintegrates. While the insert employed is not critical and those disclosed in 3,630,200 Higuchi; 3,811,444 Heller et al.; 4,177,256 Michaels et al.; 3,868,445 Ryde et al.; 3,845,201 Haddad; 3,981,303 Higuchi; and 3,867,519 Michaels, are satisfactory; in general, however, the insert described below is found preferable.

For example, one may use a solid water soluble polymer as the carrier for the medicament. The polymer used to form the insert may be any water soluble non-toxic polymer, for example, cellulose derivatives such as methylcellulose, sodium carboxymethyl cellulose, (hydroxyloweralkyl cellulose), hydroxyethyl cellulose, hydroxypropyl cellulose, hydroxypropylmethyl cellulose; acrylates such as polyacrylic acid salts, ethylacrylates, polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum, and mixtures of said polymer.

Preferably the solid insert is prepared from cellulose derivatives such as methylcellulose, hydroxyethyl cellulose, hydroxypropyl cellulose or hydroxypropylmethyl cellulose or from other synthetic materials such as polyvinyl alcohol, polyvinyl pyrrolidone, polyethylene oxide or polyvinyl methylether. Hydroxypropyl cellulose, one of the

0105996

preferred polymers for the preparation of the insert is available in several polymeric forms, all of which are suitable in the preparation of these inserts. Thus, the product sold by Hercules, Inc. of Wilmington, Delaware under the name KLUCEL such as KLUCEL HF, HWF, MF, GF, JF, LF and EF which are intended for food or pharmaceutical use are particularly useful. The molecular weight of these polymers useful for the purposes described herein may be at least 30,000 to about 1,000,000 or more. Similarly, an ethylene oxide polymer having a molecular weight of up to 5,000,000 or greater, and preferably 100,000 to 5,000,000 can be employed. Further, for example, POLYOX a polymer supplied by Union Carbide Co. may be used having a molecular weight of about 50,000 to 5,000,000 or more and preferably 3,000,000 to 4,000,000. Other specific polymers which are useful are polyvinyl pyrrolidine having a molecular weight of from about 10,000 to about 1,000,000 or more, preferably up to about 350,000 and esecially about 20,000 to 60,000; polyvinyl alcohol having a molecular weight of from about 30,000 to 1,000,000 or more, particularly about 400,000 and especially from about 100,000 to about 200,000; hydroxypropylmethyl cellulose having a molecular weight of from about 10,000 to 1,000,000 or more, particularly up to about 200,000 and especially about 80,000 to about 125,000; methyl celluloe having a molecular weight of from about 10,000 to about 1,000,000 or more, preferably up to about 200,000 and especially about 50 to 100,000; and CARBOPOL (carboxyvinyl polymer) of B. F. Goodrich and Co.

designated as grades 934, 940 and 941. It is clear that for the purpose of this invention the type and molecular weight of the polymer is not critical. Any water soluble polymers can be used having an average molecular weight which will afford dissolution of the polymer and accordingly the medicament in any desired length of time. The inserts, therefore, can be prepared to allow for retention and accordingly effectiveness in the eye for any desired period. The insert can be in the form of a square, rectangle, oval, circle, doughnut, semi-circle, 1/4 moon shape, and the like. Preferably the insert is in the form of a rod, doughnut, oval or 1/4 moon. The insert can be readily prepared, for example, by dissolving the medicament and the polymer in a suitable solvent and the solution evaporated to afford a thin film of the polymer which can then be subdivided to prepare inserts of appropriate size. Alternatively the insert can be prepared by warming the polymer and the medicament and the resulting mixture molded to form a thin film. Preferably, the inserts are prepared by molding or extrusion procedures well known in the art. The molded or extruded product can then be subdivided to afford inserts of suitable size for administration in the eye. The insert can be of any suitable size readily fit into the eye. For example, castings or compression molded films having a thickness of about 0.25 mm. to 15.0 mm. can be subdivided to obtain suitable inserts. Rectangular segments of the cast or compressed film having a thickness between about 0.5 and 1.5 mm. can be cut to afford shapes such as rectangular plates of 4 x 5-20 mm. or ovals of comparable size. Similarly, extruded rods having a diameter between about 0.5 and 1.5 mm. can be cut into suitable sections to provide the desired amount of polymer. For example, rods of 1.0 to 1.5 mm. in diameter and about 20 mm. long are found to be satisfactory. The inserts may also be directly formed by injection molding. It is preferred that the ophthalmic inserts containing the medicament of the present invention be formed so that they are smooth and do not have any sharp edges or corners which could cause damage to the eye. Since the term smooth and sharp edges or corners are subjective terms, in this application these terms used to indicate that excessive irritation of the eye will not result from the use of the insert.

The ocular medicinal inserts can also contain plasticizers, buffering agents and preservatives. Plasticizers suitable for this purpose must, of course, also be completely soluble in the lacrimal fluids of the eye. Examples of suitable plasticizers that might be mentioned are water, polyethylene glycol, propylene glycol, glycerine, trimethylol propane, di and tripropylene glycol, hydroxypropyl sucrose and the like. Typically, such plasticizers can be present in the ophthalmic insert in an amount ranging from up to about 30% by weight. A particularly preferred plasticizer is water which is present in amounts of at least about 5% up to 40%. In actual practice, a water content of from about 10% to about 20% is preferred since it may be easily accomplished and adds the desired softness and pliability to the insert.

When plasticizing the solid medicinal product with water, the product is contacted with air having a relative humidity of at least 40% until said product picks up at least about 5% water and becomes softer and more pliable. In a preferred embodiment, the relative humidity of the air is from about 60% to about 99% and the contacting is continued until the water is present in the product in amounts of from about 10% to about 20%.

Suitable water soluble preservatives which may be employed in the insert are sodium bisulfate, sodium thiosulfate, ascorbate, benzalkonium chloride, chlorobutanol, thimerosal, phenylmercuric acetate, phenylmercuric borate, parabens, benzyl alcohol and phenylethanol. These agents may be present in amounts of from 0.001 to 5% by weight of solid insert, and preferably 0.1 to 2%.

Suitable water soluble buffering agents are alkali, alkali earth carbonates, phosphates, bicarbonates, citrates, borates, and the like, such as sodium phosphate, citrate, borate, acetate, bicarbonate and carbonate. These agents may be present in amounts sufficient to obtain a pH of the system of between 5.5 to 8.0 and especially 7-8; usually up to about 2% by weight of polymer. The insert may contain fom about 1 mg. to 100 mg. of water soluble polymer, more particularly fom 5 to 50 mg. and especially from 5 to 20 mg. The medicament is present from about 0.1 to about 25% by weight of insert.

The hydrogen maleate compound has been studied with respect to its ability to lower intraocular pressure of normal rabbits and rabbits with experimental hypertension. This study demonstrated that the compound is very effective in lowering intraocular pressure after topical application. Pressure was reduced in the normal and the hypertensive eye.

The following examples are given by way of illustration.

0105996

- 14 -          16618

## EXAMPLE 1

A.  Preparation of R,S-1-t-butylamino-2,3,dihydroxypropane.

A solution of R,S-glycidol (105 g; 1.42 moles) in 100 ml of isopropanol is added dropwise over one hour to a solution of t-butylamine (197 g; 2.7 moles) in 200 ml isopropanol while maintaining the temperature between 46° - 70° C.  The solution is aged at 70°C for one hour and the excess t-butylamine is recovered by atmospheric distillation.  The distillation is continued until the pot temperature reaches 110°C.  Acetone (700 ml) is then added to the residue and the temperature of the final solution adjusted to 40°-45°C.

B.  Resolution of R,S-1-t-butylamino-2,3-dihydroxypropane.

To the final solution from (A) is added 83.0 g (0.645 moles) of R-pyroglutamic acid (97% pure) and the resultant solution mixture is refluxed, with stirring, for 1.5 hours.  This solution is then cooled to room temperature over 2.5 hours, with stirring.

The R-pyroglutamic acid. R-1-t-butylamino-2,3-dihydroxypropane diastereoisomer which separates from the solution is filtered off and washed with 2 X 50 ml of acetone.

C.  Regeneration of R-1-t-butylamine-2,3-dihydroxypropane.

The R-1-t-butylamino-2,3-dihydroxypropane is regenerated from the (B) diastereoisomer by dissolving the diastereoisomer in 200 ml of water and passing the solution through a column of 350 ml of IR-120 ($H^+$). IR-120 ($H^+$) is a gelular, strongly acidic, cation exchange resin marketed by Rohn & Haas Company. The column is washed with water until a negative test for pyroglutamic acid is obtained. R-pyroglutamic acid was recovered, in excess of 95% yield, by concentrating to dryness, slurrying the residue with isopropanol and filtering off the R-pyroglutamic acid.

The R-1-t-butylamino-2,3-dihydroxypropane is eluted from the IR-120 resin by washing with 5% ammonium hydroxide solution. The eluate is concentrated to dryness and the residue recrystallized from 150 ml of xylene to give pure R-1-t-butylamino-2,3-dihydroxypropane, (R(+)-glycolamine).

### EXAMPLE 2

Step A. - Preparation of R-2-phenyl-3-tert-butyl-5-hydroxymethyloxazolidine

A mixture of R(+)-glycolamine (20 g.; 0.136 mole), benzaldehyde (50 ml.; 288 mmole) and benzene (30 ml.) is heated under reflux for 8 hours while removing the water as formed into a Dean Stark trap filled with benzene. The temperature of the reaction mixture is maintained at 110-113°C. over the entire period. The benzene is removed in vacuo (15 mm.

0105996

- 16 -                           16618

pressure) and the excess benzaldehyde is removed by distillation at 0.1 mm. pressure. The residual oil R-2-phenyl-3-tert-butyl-5-hydroxymethyloxazoline can be used directly in the next step. If desired, the oxazolidine can be distilled to provide a product of higher purity.

Step B. - Preparation of R(+) - 3-morpholino -4 - (3-tert - butylamino - 2 - hydroxy-propoxy) - 1,2,5 - thiadiazole and its hydrogen maloate salt.

A mixture of 3-morpholino-4-chloro-1,2,5-thiadiazole (2.05 g.; 10 mmole), R- 2 - phenyl - 3 - tert-butyl-5-hydroxymethyl oxazolidine (10 mmole) and potassium tert-butoxide in tert-butanol (11.7 ml. of 0.885 N, 10 mmole) is stirred at 25° C. for 16 hours. The solvent then is evaporated in vacuo and the residue treated with 20 ml. of 1 N hydrochloric acid. The mixture is heated at 65° C. for one-half hour, cooled to 25° C. and extracted with ether. The aqueous layer is made alkaline with potassium carbonate and extracted with ether. The extracts are washed with water, dried and evaporated to an oily residue of R (+)-3 - morpholino - 4 - (3 - tert-butylamino-2-hydroxypropoxy)-1,2,5-thiadiazole. This oil is dissolved in 50 ml. of tetrahydrofuran, treated with charcoal (1.5 g.), filtered, and the cake washed with 20 ml. of tetrahydrofuran. To this solution is added maleic acid [5.0 g.; 1 mole equivalent per mole of R(+)-3-morpholino - 4 - (3 - tert-butylamino-2-hydroxy-propoxy) - 1,2,5 - thiadiazole] dissolved in tetrahydrofuran (25 ml.). The mixture then is seeded and aged one hour at 25° C. The crystallized hydrogen maleate salt is separated by filtration, washed with tetrahydrofuran and dried at 50° C. in vacuo to give R(+)-3-morpholino - 4 - (3-tert-butylamino-2-hydroxy-propoxy)-1,2,5-thiadiazole hydrogen maleate.

By replacing the maleic acid employed in the above procedure by hydrochloric acid, sulfuric acid, tartaric acid or any other desired acid the corresponding acid salt is formed.

## EXAMPLE 3

### Solution Composition

| | | |
|---|---|---|
| R-(+)-1-tert-butylamino-3-[(4-morpholino-1,2,5-thiadiazol-3-yl)oxy]-2-propanyl hydrogen maleate (I) | 1 mg. | 15 mg. |
| Sodium phosphate monobasic .2H$_2$O | 9.38 mg. | 6.10 mg. |
| Dibasic sodium phosphate .12H$_2$O | 28.48 mg. | 16.80 mg. |
| Benzalkonium chloride | 0.10 mg. | 0.10 mg. |
| Sodium hydroxide q.s. | pH 6.8 | pH 6.8 |
| Water for injection q.s. ad. | 1.0 ml. | 1.0 ml. |

(I), phosphate buffer salts, and benzalkonium chloride are added to and dissolved in water. The pH of the solution is adjusted to 6.8 with sodium hydroxide and the final solution diluted to volume. The solution is rendered sterile by filtration through a sterilizing filter.

0105996

## EXAMPLE 4

R-(+)-1-tert-butylamino-3-[(4-morpholino-
1,2,5-thiadiazol-3-yl)oxy]-2-
propanol hydrogen maleate (I)    5 mg.

petrolatum q.s. ad.    1 gram

Compound (I) and the petrolatum are aseptically combined.

## EXAMPLE 5

R-(+)-1-tert-butylamino-3-[(4-morpholino-
1,2,5-thiadiazol-3-yl)oxy]-2-
propanol hydrogen maleate (I)    1 mg.

Hydroxypropylcellulose q.s.    12 mg.

Ophthalmic inserts are manufacture from compression molded films which are prepared on a Carver Press by subjecting the powder mixture of the above ingredients to a compressional force of 12,000 lbs. (guage) at 300°F for one to four minutes. The film is cooled under pressure by having cold water circulate in the platen. Ophthalmic inserts are then individually cut from the film with a rod-shaped punch. Each insert is placed into a vial, which is then placed in a humidity cabinet (88% R.H. at 30°C) for two to four days. After removal from the humidity cabinet, the vials are stoppered and then capped. The vials containing the hydrate insert are then autoclaved at 250°F for 1/2 hour.

## EXAMPLE 6

R-(+)-1-tert-butylamino-3-[(4-morpholino-
1,2,5-thiadiazol-3-yl)oxy]-2-
propanyl hydrogen maleate (I)               1 mg.

Hydroxypropyl cellulose q.s. ad.           12 mg.

Ophthalmic inserts are manufactured from a solvent cast film prepared by making a viscous solution of the powder using methanol as the solvent. The solution is placed on a Teflon plate and allowed to dry at ambient conditions. After drying, the film is placed in an 88% R. H. cabinet until it is pliable. Appropriately sized inserts are cut from the film.

## EXAMPLE 7

R-(+)-1-tert-butylamino-3-[(4-morpholino-
1,2,5-thiadiazol-3-yl)oxy]-2-
propanyl hydrogen maleate (I)               1 mg.

Hydroxypropyl methyl cellulose q.s. ad.    12 mg.

Ophthalmic inserts are manufactured from a solvent cast film which is prepared by making a viscous solution of the powder blend using a methanol/water solvent system (10 ml. methanol is added to 2.5 g. of powder blend, to which 11 ml. of water (in three divided portions) is added. The solution is placed on a Teflon plate and allowed to dry at ambient conditions. After drying, the film is placed in an 88% R. H. cabinet until it is pliable. Appropriately sized inserts are then cut from the film.

EXAMPLE 8

R-(+)-1-tert-butylamino-3-[(4-morpholino-
1,2,5-thiadiazol-3-yl)oxy]-2-
propanyl hydrogen maleate (I)                    1 mg.

Hydroxypropylmethyl cellulose q.s. ad.        12 mg.

Other β-adrenergic blocking agents enriched in the S isomer with respect to the R can be employed in the above examples in place of R-(+)-1-tert-butylamino-3-[(4-morpholino-1,2,5-thiadiazol-3-yl)oxy]-2-propanyl hydrogen maleate.

Ophthalmic inserts are manufactured from compression molded films which are prepared on a Carver Press by subjecting the powder mixture of the above ingredients to a compressional force of 12,000 lbs. (guage) at 350°F for one minute. The film is cooled under pressure by having cold water circulate in the platen. Ophthalmic inserts are then individually cut from the film with a punch. Each insert is placed into a vial, which is then placed in a humidity cabinet (88% R. H. at 30°C) for two to four days. After removal from the humidity cabinet, the vials are stoppered and then capped. The vials containing the hydrated insert are then autoclaved at 250°F for one-half hour.

It is highly preferred that the solid inserts of this invention are available for use by the patient in a pathogen free condition. Thus, it is preferred to sterilize the inserts and so as insure against recontamination, the sterilization is preferably conducted after packaging. The best mode of sterilizing is to employ ionizing irradiation including irradiation emanating from Cobalt 60 or high energy electron beams.

After packaging a convenient quantity of inserts, usually a single dose, the package is exposed to a sterilizing quantity of radiation. The preferred packaging employs sealing the inserts between layers of film or foil and then sealing or laminating the layers together about the edges. The techniques for performing the sterilization are well known and accepted, for example, as outlined in International Atomic Energy Commission, Code of Practice for Radiosterilization of Medical Products, 1967, pp. 423-431; and Block, Disinfection, Sterilization and Preservation, 2nd Ed., Lea & Febiger, Philadelphia, 1977, pp. 542-561.

The required quantity of irradiation can be determined experimentally by testing irradiated inserts for viable bacteria. Generally, the amount of irradiation desired to achieve sterilization is defined by the $D_{10}$ value. The $D_{10}$ value is the radiation dose that will reduce a given population of organisms by a factor of 10. Based on $D_{10}$ values, experimentally obtained for Bacillus pumilus, and presterilization contamination levels, a dose of 1.36 megarads is effective in obtaining a sterile product.

0105996

WHAT IS CLAIMED IS:

1. A method for treating glaucoma, ocular hypertension or for lowering intraocular pressure which comprises topically applying to an affected eye an intraocular pressure lowering effective amount of a ß-adrenergic blocking agent where the ß-adrenergic blocking agent is selected from the group methypranol; propranolol; alprenolol; oxprenolol; practolol; acebutolol; prindolol; metoprolol; atenolol; sotalol; metindol; nadolol; labetalol; practolol; bunitrolol; RMI 81968 (medroxalol); tiprenolol; tolamolol; celiprolol; mepindolol; indenolol; bupranolol; penbutolol; trimepranol; 2-(3-(1,1-dimethylethyl)-amino-2-hydroxypropoxy)-3-pyridenecarbonitrile hydrochloride; 1-tertbutylamino-3-(2,5-dichloro-phenoxy)-2-propanol; 1-isopropylamino-3-[4-(2-cyclo-propylmethoxy-ethyl)phenoxy]-2-propanol; 3-isopropyl-amino-1-(7-methylindan-4-yloxy)-2-butanol; 2-(3-tert-butylamino-2-hydroxypropylthio)-4-(5-carbamoyl-2-thienyl)thiazole, and 7-(2-hydroxy-3-tert-butylamino-propoxy)-phthalide, wherein the R stereoisomer thereof is enriched with respect to the S stereoisomer of said ß-adrenergic blocking agent.

2. A method according to Claim 1 wherein the ß-adrenergic blocking agent is administered as 0.01 to 5% a solution of said agent in an ophthalmologically acceptable carrier.

3. An ophthalmic composition for the topical treatment of glaucoma and ocular hypertension

0105996

- 23 -                                      16618Y

comprising in solution an intraocular pressure lowering effective amount of a ß-adrenergic blocking agent selected from the group methypranol; propranolol; alprenolol; oxprenolol; practolol; acebutolol; prindolol; metoprolol; atenolol; sotalol; metindol; nadolol; labetalol; practolol; bunitrolol; RMI 81968 (medroxalol); tiprenolol; tolamolol; celiprolol; mepindolol; indenolol; bupranolol; penbutolol; trimepranol; (R)-2-(3-(1,1-dimethylethyl)-amino-2-hydroxypropoxy)-3-pyridenecarbonitrile hydrochloride; 1-tertbutylamino-3-(2,5-dichloro-phenoxy)-2-propanol; 1-isopropylamino-3-[4-(2-cyclo-propylmethoxy-ethyl)phenoxy]-2-propanol; 3-isopropyl-amino-1-(7-methylindan-4-yloxy)-2-butanol; 2-(3-tert-butylamino-2-hydroxypropylthio)-4-(5-carbamoyl-2-thienyl)thiazole, and 7-(2-hydroxy-3-tert-butylamino-propoxy)-phthalide, where the R isomer of said ß-adrenergic blocking agent is enriched with respect to the S isomer.

4.  A composition according to Claim 1 wherein the solution is from 0.01 to 5% ß-adrenergic blocking agent.

5.  A composition according to Claim 1 wherein the solution is from 0.5 to 2% ß-adrenergic blocking agent.

6.  A method for treating glaucoma and ocular hypertension and for lowering intraocular pressure which comprises topically applying to an affected eye an intraocular pressure lowering effective amount of an R stereoisomer free of S

stereoisomer of a ß-adrenergic blocking agent, selected from the group methylpranol; propranolol; alprenolol; oxprenolol; practolol; acebutolol; prindolol; metoprolol; atenolol; gutolol; metindol; nadolol; labetalol; practolol; bunitrolol; RMI 81968 (medroxalol); SL 75212; ICI 118551; tiprenolol; tolamolol; celiprolol; mepindolol; indenolol; bupranolol; penbuolol; trimepranol; GYKI 41099; DL 071-IT, BFE-60; S-596, and MK-761.

7.   A method according to Claim 6 wherein the ß-blocking agent is administered in a water soluble polymeric insert.

8.   A method according to Claim 6 wherein the ß-blocking agent is administered as 0.01 to 5% a solution of said agent in an ophthalmologically acceptable carrier.

9.   An ophthalmic composition for the topical treatment of glaucoma and ocular hypertension comprising in solution an intraocular pressure lowering effective amount of an R stereoisomer free of S stereoisomer of a ß-adrenergic blocking agent selected from the group methypranol; propranolol; alprenolol; oxprenolol; practolol; acebutolol; prindolol; metoprolol; atenolol; sotalol; metindol; nadolol; labetalol; practolol; bunitrolol; RMI 81968 (medroxalol); tiprenolol; tolamolol; celiprolol; mepindolol; indenolol; bupranolol; penbutolol; trimepranol; (R)-2-(3-(1,1-dimethylethyl)-amino-2-hydroxypropoxy)-3-pyridenecarbonitrile hydrochloride; 1-tertbutylamino-3-(2,5-dichloro-

phenoxy)-2-propanol; 1-isopropylamino-3-[4-(2-cyclopropylmethoxy-ethyl)phenoxy]-2-propanol; 3-isopropylamino-1-(7-methylindan-4-yloxy)-2-butanol; 2-(3-tert-butylamino-2-hydroxypropylthio)-4-(5-carbamoyl-2-thienyl)thiazole, and 7-(2-hydroxy-3-tert-butylaminopropoxy)-phthalide.

10.  A composition according to Claim 9 wherein the solution is from 0.01 to 5% of said ß-blocking agent.

0105996

0105996
16618Y

WHAT IS CLAIMED IS :

ACCEPTED
CLAIMS            25 OCT. 1983

1. An ophtalmic composition for treating glaucoma, ocular hypertension or for lowering intraocular pressure wich comprises an intraocular pressure lowering effective amount of a $\beta$-adrenergic blocking agent where the $\beta$-adrenergic blocking agent is selected from the group methypranol, propranolol, alprenolol; oxprenolol, acebutolol, prindolol; metoprolol; atenolol; sotalol; metindol, nadolol;                   practolol; bunitrolol; RMI 81968 (medroxalol); tiprenolol; tolamolol; celiprolol; mepindolol, indenolol; bupranolol; penbutolol; trimepranol; 2-(3-(1,1-dimethylethyl)-amino-2-hydroxypropoxy)-3-pyridenecarbonitrile hydrochloride; 1-tertbutylamino-3-(2,5-dichlorophenoxy)-2-propanol; 1-isopropylamino-3-[4-(2-cyclopropylmethoxy-ethyl)phenoxy]-2-propanol; 3-isopropyl-amino-1-(7-methylindan-4-yloxy)-2-butanol; 2-(3-tertbutyla-mino-2-hydroxypropylthio)-4-(5-carbamoyl-2-thienyl) thiazole, and 7-(2-hydroxy-3-tert-butylaminopropoxy)-phthalide, and a carrier suitable for topical administration in the eye, characterized in that said $\beta$-adrenergic blocking agent is under the form of the R stereoisomer thereof enriched with respect to the S stereoisomer.

2. An ophtalmic composition according to claim 1 characterized in that the $\beta$-adrenergic blocking agent compri-ses at least 85 % by weight of R isomer based on the weight of both isomers.

3. An ophtalmic composition according to claim 2 characterized in that the $\beta$-adrenergic blocking agent compri-ses at least 90 % by weight of the R isomer based on the weight of both isomers.

4. An ophtalmic composition according to any one of claims 1 to 3 characterized in that the ophtalmic composition comprises from 0,01 to 5 % by weight of the $\beta$-adrener-gic blocking agent.

23 0105996

5. An opthalmic composition according to claim 4 characterized in that the ophtalmic composition comprises from 0,5 to 2 % by weight of the $\beta$-adrenergic blocking agent.

6. An ophtalmic composition according to any one of claims 1 to 5 characterized in that $\beta$-adrenergic blocking agent is in solution.

7. An ophtalmic composition according to any one of claims 1 to 5 ,characterized in that the suitable carrier is an ophtalmic insert.

8. An ophtalmic composition according to claim 7 characterized in that the ophtalmic insert is a water soluble polymeric insert.

25 OCT. 1983

0105996

AMENDED
CLAIMS

25 OCT. 1983

CLAIMS FOR AUSTRIA :

1. Process for preparation of an ophtalmic composition for treating glaucoma, ocular hypertension or for lowering intraocular pressure wich comprises mixing an intraocular pressure lowering effective amount of a $\beta$-adrenergic blocking agent where the $\beta$-adrenergic blocking agent is selected from the group methypranol; propranolol; alprenolol, oxprenolol; practolol; acebutolol; prindolol; metoprolol; atenolol; sotalol; metindol; nadolol; bunitrolol; RMI 81968 (medroxalol); tiprenolol; tolamolol; celiprolol; mepindolol; indenolol; bupranolol; penbutolol; trimepranol; 2-(3-(1,1-dimethylethyl)- amino-2-hydroxypropoxy) -3-pyridenecarbonitrile; hydrochloride; 1-tertbutylamino-3- (2,5-dichlorophenoxy)-2-propanol; 1-isopropylamino-3-/4- (2-cyclopropylmethoxy-ethyl)phenoxy7-2-propanol; 3-isopropyl- amino-1-(7-methylindan-4-yloxy)-2-butanol; 2-(3-tertbutylamino- 2-hydroxypropylthio)-4-(5carbamoyl-2-thienyl-thiazole, ans 7-(2-hydroxy-3-tert-butylamino-propoxy)-phthalide and a carrier suitable for topical administration in the eye, characterized in that said $\beta$-adrenergic blocking agent is under the form of the R stereoisomer thereof enriched with respect to the S stereoisomer.

2. Process for preparation of an ophtalmic composition according to claim 1 characterized in that the $\beta$-adrenergic blocking agent comprises at least 85 % by weight of R isomer based on the weight of both isomers.

3. Process for preparation of an ophtalmic composition according to claim 1 characterized in that the $\beta$-adrenergic blocking agent comprises at least 90 % by weight of the R isomer based on the weight of both isomers.

4. Process for preparation of an ophtalmic composition according to claim 1 characterized in that the ophtalmic composition comprises from 0,01 to 5 % by weight of the $\beta$-adrenergic blocking agent.

5. Process for preparation of an ophtalmic composition according to claim 1 characterized in that the ophtalmic composition comprises from 0,5 to 2 % by weight of the $\beta$-adrenergic blocking agent.

6. Process for preparation of an ophtalmic composition according to claim 1 characterized in that $\beta$-adrenercig blocking agent is in solution.

7. Process for preparation of an ophtalmic composition according to claim 1 characterized in that the suitable carrier is an ophtalmic insert.

8. Process for preparation of an ophtalmic composition according to claim 1 characterized in that the ophtalmic insert is a water soluble polymeric insert.

## PARTIAL EUROPEAN SEARCH REPORT

**European Patent Office**

which under Rule 45 of the European Patent Convention
shall be considered, for the purposes of subsequent
proceedings, as the European search report

Application number

| | DOCUMENTS CONSIDERED TO BE RELEVANT | | EP 82 40 1896 |
|---|---|---|---|

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol.97, no.15, October 11, 1982, abstract no. 120967v COLUMBUS OHIO (US) & Graefe's Arch. Clin. Exp. Ophthalmol. 1982, 218(6), 297-300 H.C. INNEMEE et al.: "The role of $\beta$2-adrenoceptors in the IOP-lowering effect of adrenaline." <br><br> * the whole document * <br><br> -- | 3-5,9, 10 | A 61 K 31/13 <br> A 61 K 31/41 |
| X | GB - A - 1 253 709 (CH. E. FROSST & CO.) <br><br> * page 1, line 6 - page 3, line 3; example 1A,1B,16, 16 Section 15, 16 * <br><br> -- <br><br> ./. | 3-5,9, 10 | |

TECHNICAL FIELDS
SEARCHED (Int. Cl. 3)

A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 3-5,9,10
Claims searched incompletely:
Claims not searched: 1,2,6-8
Reason for the limitation of the search:

Method for treatment of the human or animal body by surgery or therapy (see article 52(4) of the European Patent. Convention).

| Place of search | Date of completion of the search | Examiner |
|---|---|---|
| The Hague | 08-06-1983 | PAUWELS |

European Patent Office

**PARTIAL EUROPEAN SEARCH REPORT**

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| X | CHEMICAL ABSTRACTS, vol.91, 27-08-1979 – 10-09-1979, page 48, abstract no.68523g COLUMBUS OHIO (US) & Albrecht von Graefes Arch. Klin. Exp. Ophtalmol., 1979, 210(1), 1-8 L. BONOMI et al.: "Comparison of the effects of nine beta-adrenergic blocking agents on intraocular pressure in rabbits." <br><br> * the whole document * | 3-5,9, 10 | TECHNICAL FIELDS SEARCHED (Int. Cl.³) |

------